(19) Europäisches Patentamt / European Patent Office / Office européen des brevets

(11) **EP 3 960 720 A1**

(12) **EUROPEAN PATENT APPLICATION**
published in accordance with Art. 153(4) EPC

(43) Date of publication:
**02.03.2022 Bulletin 2022/09**

(21) Application number: **20795809.1**

(22) Date of filing: **24.04.2020**

(51) International Patent Classification (IPC):
**C04B 35/48** (2006.01)   **A61K 6/80** (2020.01)
**A61K 6/84** (2020.01)

(52) Cooperative Patent Classification (CPC):
**A61K 6/80; A61K 6/84; C04B 35/48**

(86) International application number:
**PCT/JP2020/017773**

(87) International publication number:
**WO 2020/218541 (29.10.2020 Gazette 2020/44)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **25.04.2019 JP 2019084329**

(71) Applicant: **Kuraray Noritake Dental Inc.
Kurashiki-shi, Okayama 710-0801 (JP)**

(72) Inventors:
• **SUZUKI, Ichiro
  Miyoshi-shi, Aichi 470-0293 (JP)**
• **KATO, Shinichiro
  Miyoshi-shi, Aichi 470-0293 (JP)**

(74) Representative: **D Young & Co LLP
120 Holborn
London EC1N 2DY (GB)**

(54) **ZIRCONIA CALCINED BODY SUITABLE FOR DENTAL USE AND METHOD FOR MANUFACTURING SAME**

(57) The present invention provides a zirconia pre-sintered body that enables one visit treatment due to the short firing time and from which a zirconia sintered body having excellent translucency is obtained irrespective of the thickness, and a method for producing the zirconia pre-sintered body. The present invention is a method for producing a zirconia molded body, wherein the zirconia molded body comprises: zirconia; and a stabilizer capable of inhibiting a phase transformation of zirconia, at least a part of the stabilizer is undissolved in zirconia as a solid solution, and the method comprises press molding a mixed powder comprising zirconia and the stabilizer at a pressure of 175 MPa or more to obtain a zirconia molded body.

# EP 3 960 720 A1

**Description**

TECHNICAL FIELD

The present invention relates to a zirconia pre-sintered body and a method for producing the same. The present invention also relates to a dental material comprising the zirconia pre-sintered body, and a method for producing a zirconia molded body for producing the zirconia pre-sintered body.

BACKGROUND ART

Zirconia is a compound that undergoes a phase transformation between crystal systems. Partially stabilized zirconia (PSZ) and fully stabilized zirconia, which are used in a wide variety of fields, inhibit such phase transformations with a stabilizer, such as yttria (yttrium oxide; $Y_2O_3$), dissolved in zirconia as a solid solution.

In dentistry, zirconia materials have been used mostly in frame applications because of their low translucency, though zirconia materials are high in strength. The improved translucency of more recent zirconia materials has prompted fabrication of dental prostheses solely made of zirconia. Patent Literature 1 discloses a high-translucency colored zirconia sintered body suited for dental use (particularly for front teeth).

Traditionally, fabrication of all-zirconia dental prostheses is usually performed at dental laboratories. However, it is becoming increasing popular to more conveniently make such dental prostheses at the dental clinic. In this case, firing of zirconia needs to be completed in a short time period.

Furthermore, since zirconia has high strength, it is expected that zirconia can be used in many cases, such as cases in which strength is required not only for single crowns but also for continuous crowns and bridges.

Patent Literature 1 discloses that for many cases such as inlays and bridges, firing conditions are determined by changes in various factors such as the maximum lateral wall thickness, the maximum occlusal wall thickness, and the maximum cross-sectional area of a prosthesis.

Patent Literature 2 discloses a zirconia sintered body having uniform translucency to the inside when firing is performed at a temperature increase rate of 5°C/min or less and a maximum retention temperature of 1550°C or more.

CITATION LIST

Patent Literature

Patent Literature 1: JP 2016-540562 A
Patent Literature 2: JP 2017-128466 A

SUMMARY OF THE INVENTION

Technical Problem

In the method described in Patent Literature 1, complicated calculations are performed by a computer and this does not clarify a prosthesis manufacturing time, causing a possibility that one visit treatment cannot be achieved.

In the method of Patent Literature 2, firing requires 10 hours or more, and thus one visit treatment cannot be achieved. Accordingly, a zirconia pre-sintered body is needed that can be fired into a sintered body that develops the shade suited for dental use (particularly, at the dental clinic), even with a short firing time. There is also a need for a zirconia pre-sintered body that can be fired into a sintered body with maintained translucency even when the firing time is short. Furthermore, a zirconia pre-sintered body fired in a short time into a sintered body having a large thickness (for example, 10 mm or more) might have a deteriorated translucency. Accordingly, there is a need for a zirconia pre-sintered body that can be fired into a sintered body in which deterioration in translucency is reduced even when the firing time is short and the thickness of the sintered body after firing is 10 mm or more.

In view of this, the present invention aims to provide a zirconia pre-sintered body that enables one visit treatment due to the short firing time and from which a zirconia sintered body having excellent translucency is obtained irrespective of the thickness, and a method for producing the zirconia pre-sintered body.

Solution to Problem

The present inventors conducted intensive studies to find a solution to the above problems, and found that the problems can be solved by producing a zirconia pre-sintered body using a zirconia molded body produced by a specific production method or by producing a zirconia pre-sintered body having a specific bulk density at a thickness of 10 mm or more. The present invention was completed after further studies based on this finding.

Specifically, the present invention includes the following.

[1] A method for producing a zirconia molded body, wherein
the zirconia molded body comprises:

zirconia; and
a stabilizer capable of inhibiting a phase transformation of zirconia,
at least a part of the stabilizer is undissolved in zirconia as a solid solution, and the method comprises
press molding a mixed powder comprising zirconia and the stabilizer at a pressure of 175 MPa or more to obtain
a zirconia molded body.

[2] The method according to [1], wherein the stabilizer comprises yttria.
[3] The method according to [2], wherein a percentage presence $f_y$ of the yttria undissolved in the zirconia as a solid solution as calculated from a mathematical expression (1) below is more than 0%,

$$f_y(\%) = \frac{I_y(111)}{I_y(111) + I_m(111) + I_m(11-1) + I_t(111) + I_c(111)} \times 100 \quad (1)$$

where $I_y(111)$ represents a peak intensity of a (111) plane of yttria near $2\theta = 29°$ in an X-ray diffraction pattern using CuK$\alpha$ radiation, $I_m(111)$ and $I_m(11-1)$ represent peak intensities of a (111) plane and a (11-1) plane, respectively, of a monoclinic crystal system of zirconia in the X-ray diffraction pattern, $I_t(111)$ represents a peak intensity of a (111) plane of a tetragonal crystal system of zirconia in the X-ray diffraction pattern, and $I_c(111)$ represents a peak intensity of a (111) plane of a cubic crystal system of zirconia in the X-ray diffraction pattern.
[4] The method according to any one of [1] to [3], wherein the pressure in the press molding is 200 MPa or more.
[5] The method according to any one of [1] to [4], wherein the zirconia is predominantly monoclinic.
[6] The method according to [5], wherein a fraction $f_m$ of a monoclinic crystal system of zirconia is 55% or more as calculated from a mathematical expression (2) below,

$$f_m(\%) = \frac{I_m(111) + I_m(11-1)}{I_m(111) + I_m(11-1) + I_t(111) + I_c(111)} \times 100 \quad (2)$$

where $I_m(111)$ and $I_m(11-1)$ represent peak intensities of a (111) plane a (11-1) plane, respectively, of a monoclinic crystal system of zirconia, $I_t(111)$ represents a peak intensity of a (111) plane of a tetragonal crystal system of zirconia, and $I_c(111)$ represents a peak intensity of a (111) plane of a cubic crystal system of zirconia.
[7] A method for producing a zirconia pre-sintered body, the method comprising
firing a zirconia molded body obtained by the method according to any one of [1] to [6] at 800 to 1200°C.
[8] A zirconia pre-sintered body comprising:

zirconia; and
a stabilizer capable of inhibiting a phase transformation of zirconia, wherein
with respect to a first sintered body having a thickness of 1 mm and a second sintered body having a thickness
of 10 mm that are each fabricated by firing the zirconia pre-sintered body for 30 minutes, a ratio of a second
translucency of a specimen having a thickness of 0.5 mm and fabricated from the second sintered body to a
first translucency of a specimen having a thickness of 0.5 mm and fabricated from the first sintered body is 90%
or more.

[9] A zirconia pre-sintered body comprising:

zirconia; and
a stabilizer capable of inhibiting a phase transformation of zirconia, wherein
at least a part of the stabilizer is undissolved in zirconia as a solid solution, and
a bulk density measured by an Archimedes method using a specimen of a pre-sintered body having a thickness
of 10 mm or more and fabricated from the zirconia pre-sintered body is 3.0 g/cm$^3$ or more.

[10] The zirconia pre-sintered body according to [8] or [9], wherein the stabilizer comprises yttria.

[11] The zirconia pre-sintered body according to [10], wherein a percentage presence $f_y$ of the yttria undissolved in the zirconia as a solid solution as calculated from a mathematical expression (1) below is more than 0%,

$$f_y(\%) = \frac{I_y(111)}{I_y(111) + I_m(111) + I_m(11-1) + I_t(111) + I_c(111)} \times 100 \qquad (1)$$

where $I_y(111)$ represents a peak intensity of a (111) plane of yttria near $2\theta = 29°$ in an X-ray diffraction pattern using CuKα radiation, $I_m(111)$ and $I_m(11-1)$ represent peak intensities of a (111) plane and a (11-1) plane, respectively, of a monoclinic crystal system of zirconia in the X-ray diffraction pattern, $I_t(111)$ represents a peak intensity of a (111) plane of a tetragonal crystal system of zirconia in the X-ray diffraction pattern, and $I_c(111)$ represents a peak intensity of a (111) plane of a cubic crystal system of zirconia in the X-ray diffraction pattern.

[12] A dental material comprising the zirconia pre-sintered body according to any one of [8] to [11].

[13] The dental material according to [12], being disc-shaped or block-shaped.

Advantageous Effects of Invention

According to the present invention, it is possible to obtain a zirconia pre-sintered body that enables one visit treatment due to the short firing time and from which a zirconia sintered body having excellent translucency is obtained irrespective of the thickness, and a method for producing the zirconia pre-sintered body. In particular, according to the present invention, it is possible to provide a zirconia pre-sintered body that can be fired into a sintered body in which deterioration in translucency is reduced even when the firing time is short and the thickness of the sintered body after firing is large (for example, 10 mm or more), and a method for producing the zirconia pre-sintered body.

By using the zirconia pre-sintered body of the present invention, it is possible to obtain a zirconia prosthesis that, for many cases, maintains translucency even when the firing conditions are not changed by geometrical features such as the maximum wall thickness, the maximum member cross-sectional area, or the volume of the prosthesis, and according to which the treatment time can be clarified before the prosthesis is fabricated and the total firing time under the firing conditions is within 30 minutes.

BRIEF DESCRIPTION OF DRAWINGS

FIG. 1 is a schematic view of a zirconia sintered body in the case where a zirconia pre-sintered body of the present invention has a multilayer structure.

FIG. 2 is an X-ray diffraction pattern of a pre-sintered body fabricated in Example 1.

FIG. 3 is an X-ray diffraction pattern of a pre-sintered body fabricated in Example 3.

FIG. 4 is an X-ray diffraction pattern of a pre-sintered body fabricated in Comparative Example 5.

DESCRIPTION OF EMBODIMENTS

A zirconia pre-sintered body of the present invention is a zirconia pre-sintered body comprising: zirconia; and a stabilizer capable of inhibiting a phase transformation of zirconia, and at least a part of the stabilizer is undissolved in zirconia as a solid solution. It is important that a bulk density measured by the Archimedes method using a specimen of a pre-sintered body having a thickness of 10 mm or more and fabricated from the zirconia pre-sintered body be 3.0 g/cm³ or more. By satisfying this condition, a zirconia sintered body having excellent translucency is obtained irrespective of the thickness with a short firing time. The bulk density is preferably 3.1 g/cm³ or more. The lower limit of the thickness of the specimen of the pre-sintered body is preferably 12 mm or more, more preferably 13 mm or more, even more preferably 14 mm or more, and particularly preferably a thickness of pre-sintered body from which a sintered body having a thickness of 10 mm or more is obtained after firing for 30 minutes. Meanwhile, the upper limit is not particularly limited, and for example, may be 40 mm or less, or 30 mm or less. The details of the method for measuring the bulk density will be described below in the EXAMPLES section. Further, the bulk density measured by the Archimedes method using a specimen of a pre-sintered body having a thickness of less than 10 mm and fabricated from the zirconia pre-sintered body is also preferably 3.0 g/cm³ or more, and more preferably 3.1 g/cm³ or more. Moreover, the ratio of the bulk density measured by the Archimedes method using the specimen of the pre-sintered body having a thickness of 10 mm or more and fabricated from the zirconia pre-sintered body to the bulk density measured by the Archimedes method using the specimen of the pre-sintered body having a thickness of less than 10 mm and fabricated from the zirconia pre-sintered body is preferably 90% or more, more preferably 95% or more, and even more preferably 100%, in view of improving translucency irrespective of the thickness.

As described above, in the zirconia pre-sintered body of the present invention, the firing time is short and the obtained zirconia sintered body has excellent translucency irrespective of the thickness. More specifically, the zirconia pre-sintered body of the present invention is a zirconia pre-sintered body comprising: zirconia; and a stabilizer capable of inhibiting a phase transformation of zirconia. With respect to a first sintered body having a thickness of 1 mm and a second sintered body having a thickness of 10 mm that are each fabricated by firing the zirconia pre-sintered body for 30 minutes, a ratio of a second translucency of a specimen having a thickness of 0.5 mm and fabricated from the second sintered body (hereinafter referred to simply as "second translucency") to a first translucency of a specimen having a thickness of 0.5 mm and fabricated from the first sintered body (hereinafter referred to simply as "first translucency") ($\Delta L_2^*/\Delta L_1^*$) is 90% or more, preferably 92% or more, and more preferably 95% or more. Also, the first translucency ($\Delta L_1^*$) and the second translucency ($\Delta L_2^*$) are preferably 10 or more, more preferably 12 or more, even more preferably 14 or more, and particularly preferably 16 or more. The details of the method for measuring the translucency will be described below in the EXAMPLES section. The firing temperature at which a zirconia pre-sintered body is fired to obtain a sintered body is, for example, preferably 1,400°C or more, more preferably 1,450°C or more. In addition, the firing temperature is, for example, preferably 1,650°C or less, and more preferably 1,600°C or less.

A zirconia pre-sintered body of the present invention will be described in detail below. The zirconia pre-sintered body is a body that can be a precursor (intermediate product) of a zirconia sintered body. In the present invention, the zirconia pre-sintered body may be, for example, a zirconia pre-sintered body that has turned into a block with incompletely sintered zirconia particles (powders).

A zirconia pre-sintered body of the present invention comprises zirconia, and a stabilizer capable of inhibiting a phase transformation of zirconia. The stabilizer is preferably one capable of forming partially stabilized zirconia. Examples of the stabilizer include oxides such as calcium oxide (CaO), magnesium oxide (MgO), yttria, cerium oxide ($CeO_2$), scandium oxide ($Sc_2O_3$), niobium oxide ($Nb_2O_5$), lanthanum oxide ($La_2O_3$), erbium oxide ($Er_2O_3$), praseodymium oxide ($Pr_6O_{11}$), samarium oxide ($Sm_2O_3$), europium oxide ($Eu_2O_3$), and thulium oxide ($Tm_2O_3$), and yttria is preferred. The content of the stabilizer in a zirconia pre-sintered body of the present invention, and the content of the stabilizer in a sintered body of a zirconia pre-sintered body of the present invention can be measured using a technique, for example, such as inductively coupled plasma (ICP) emission spectral analysis or x-ray fluorescence analysis. The content of the stabilizer in a zirconia pre-sintered body of the present invention is preferably 0.1 to 18 mol%, and more preferably 1 to 15 mol% with respect to the total mole of the zirconia and the stabilizer.

In a zirconia pre-sintered body of the present invention, the zirconia is preferably predominantly monoclinic. In the present invention, "predominantly monoclinic" means that the fraction $f_m$ of the monoclinic crystal system of zirconia is at least 50% of the total amount of all crystal systems of zirconia (the monoclinic system, the tetragonal system, and the cubic system) as calculated from a mathematical expression (2) below. In a zirconia pre-sintered body of the present invention, the fraction $f_m$ of the monoclinic system in zirconia calculated from the mathematical expression (2) below is preferably 55% or more, more preferably 60% or more, even more preferably 70% or more, yet more preferably 75% or more, particularly preferably 80% or more, more particularly preferably 85% or more, and most preferably 90% or more with respect to the total amount of the monoclinic, tetragonal, and cubic crystal systems. The fraction $f_m$ of monoclinic system can be calculated from the mathematical expression (2) below, using peaks in an X-ray diffraction (XRD) pattern by CuK$\alpha$ radiation. It is to be noted that the predominant crystal system in the zirconia pre-sintered body has possible contribution to the increased contraction temperature and the reduced firing time.

In a zirconia pre-sintered body of the present invention, the peaks of the tetragonal crystal system and the cubic crystal system may be essentially undetectable. That is, the monoclinic system may have a fraction $f_m$ of 100%.

$$f_m(\%) = \frac{I_m(111) + I_m(11-1)}{I_m(111) + I_m(11-1) + I_t(111) + I_c(111)} \times 100 \quad (2)$$

In the mathematical expression (2), $I_m(111)$ and $I_m(11-1)$ represent the peak intensities of the (111) plane and (11-1) plane, respectively, of the monoclinic crystal system of zirconia. $I_t(111)$ represents the peak intensity of the (111) plane of the tetragonal crystal system of zirconia. $I_c(111)$ represents the peak intensity of the (111) plane of the cubic crystal system of zirconia.

In a zirconia pre-sintered body of the present invention, at least a part of the stabilizer needs to be present in an undissolved form in zirconia as a solid solution. Whether or not the stabilizer is at least partly dissolved in zirconia as a solid solution can be confirmed by an XRD pattern, for example. The presence of a peak derived from the stabilizer in an XRD pattern of the zirconia pre-sintered body means that the zirconia pre-sintered body is containing a stabilizer that is not dissolved in zirconia as a solid solution. A peak derived from the stabilizer is basically not observable in an XRD pattern when the stabilizer has fully dissolved as a solid solution. It is, however, possible, depending on the crystal state or other conditions of the stabilizer, that the stabilizer may not be dissolved in zirconia as a solid solution even when the stabilizer does not produce a peak in the XRD pattern. The stabilizer can be thought of having dissolved in zirconia as a solid solution for the most part, basically completely, when zirconia is predominantly tetragonal and/or cubic, and there is no peak attributed to the stabilizer in the XRD pattern. In a zirconia pre-sintered body of the present invention, it is not required to fully dissolve the stabilizer in zirconia as a solid solution. In the present invention, "to dissolve the stabilizer as a solid solution" means that, for example, the elements (atoms) contained in the stabilizer are dissolved in zirconia as a solid solution.

In view of the strength and translucency of a zirconia sintered body fabricated from a zirconia pre-sintered body of the present invention, it is preferable to contain yttria as the stabilizer. The yttria content is preferably 3 mol% or more, more preferably 3.5 mol% or more, even more preferably 3.8 mol% or more, and particularly preferably 4.0 mol% or more, with respect to the total mole of zirconia and yttria. The translucency of the zirconia sintered body can increase with a yttria content of 3 mol% or more. Also, the yttria content is preferably 7.5 mol% or less, more preferably 7.0 mol% or less, even more preferably 6.5 mol% or less, and particularly preferably 6.0 mol% or less, with respect to the total mole of zirconia and yttria. Decrease of the strength of the zirconia sintered body can be reduced with a yttria content of 7.5 mol% or less.

In a zirconia pre-sintered body of the present invention, the percentage presence $f_y$ of yttria not dissolved in zirconia as a solid solution (hereinafter, referred to also as "undissolved yttria") can be calculated from a mathematical expression (1) below. The percentage presence $f_y$ of undissolved yttria is preferably more than 0%, more preferably 1% or more, even more preferably 2% or more, and particularly preferably 3% or more. The upper limit of the percentage presence $f_y$ of undissolved yttria may be, for example, 15% or less. However, preferably, the upper limit of the percentage presence $f_y$ of undissolved yttria depends on the yttria content of the zirconia pre-sintered body. The percentage presence $f_y$ may be 7% or less for a yttria content of 3 mol% or more and less than 4.5 mol%. The percentage presence $f_y$ may be 11% or less for a yttria content of 4.5 mol% or more and less than 5.8 mol%. The percentage presence $f_y$ may be 15% or less for a yttria content of 5.8 mol% or more and less than 7.5 mol%. In a pre-sintered body containing zirconia that is predominantly monoclinic, the percentage presence $f_y$ of undissolved yttria in the zirconia pre-sintered body is affected by the pre-sintering temperature, and is also affected by the average particle diameter of a mixed powder containing zirconia and yttria as a raw material. Due to the presence of undissolved yttria in the zirconia pre-sintered body as a solid solution, a zirconia sintered body having high translucency can be obtained even with a short firing time.

In a zirconia pre-sintered body of the present invention, the percentage presence $f_y$ is preferably 0.5% or more, more preferably 1.0% or more, and even more preferably 2.0% or more for a yttria content of 3 mol% or more and less than 4.5 mol%. The percentage presence $f_y$ is preferably 1% or more, more preferably 2% or more, and even more preferably 3% or more for a yttria content of 4.5 mol% or more and less than 5.8 mol%. The percentage presence $f_y$ is preferably 2% or more, more preferably 3% or more, and even more preferably 4% or more for a yttria content of 5.8 mol% or more and 7.5 mol% or less. In a zirconia pre-sintered body of the present invention, the ratio $f_m/f_y$ is preferably 20 to 200, more preferably 25 to 100, and even more preferably 30 to 60 for a yttria content of 3 mol% or more and less than 4.5 mol%. The ratio $f_m/f_y$ is preferably 5 to 45, more preferably 10 to 40, and even more preferably 15 to 35 for a yttria content of 4.5 mol% or more and less than 5.8 mol%. The ratio $f_m/f_y$ is preferably 2 to 40, more preferably 5 to 35, and even more preferably 10 to 30 for a yttria content of 5.8 mol% or more and 7.5 mol% or less.

$$f_y(\%) = \frac{I_y(111)}{I_y(111) + I_m(111) + I_m(11-1) + I_t(111) + I_c(111)} \times 100 \qquad (1)$$

In the mathematical expression (1), $I_y(111)$ represents the peak intensity of the (111) plane of yttria near $2\theta = 29°$ in an XRD pattern using CuK$\alpha$ radiation. $I_m(111)$ and $I_m(11-1)$ represent the peak intensities of the (111) plane and (11-1) plane, respectively, of the monoclinic crystal system of zirconia. $I_t(111)$ represents the peak intensity of the (111) plane of the tetragonal crystal system of zirconia. $I_c(111)$ represents the peak intensity of the (111) plane of the cubic crystal system of zirconia.

The mathematical expression (1) is also applicable to calculations of the percentage presence of undissolved stabilizers as a solid solution other than yttria by substituting other peaks for $I_y(111)$.

In order to ensure the strength needed for mechanical working, a zirconia pre-sintered body of the present invention has a flexural strength of preferably 15 MPa or more. For ease of mechanical working, the flexural strength of the pre-sintered body is preferably 70 MPa or less, and more preferably 60 MPa or less.

The flexural strength can be measured in compliance with ISO 6872:2015 (Dentistry-Ceramic materials), and the measurement is made using the same conditions, except for the specimen size, specifically, by using a specimen measuring 5 mm × 10 mm × 50 mm in size. The surface of the specimen, and the C surface (a surface created by chamfering a corner of the specimen at a 45° angle) are longitudinally finished with #600 sandpaper. The specimen is disposed in such an orientation that the widest surface is vertically situated (loading direction). In the three-point flexural measurement, the distance between supports (span) is 30 mm, and the crosshead speed is 0.5 mm/min.

A zirconia pre-sintered body of the present invention may comprise an additive or additives other than zirconia and the stabilizer, provided that the present invention can exhibit its effects. Examples of such additives include colorants (including pigments, complex pigments, and fluorescent agents), alumina ($Al_2O_3$), titanium oxide ($TiO_2$), and silica ($SiO_2$).

Examples of the pigments include oxides of at least one element selected from the group consisting of Ti, V, Cr, Mn, Fe, Co, Ni, Zn, Y, Zr, Sn, Sb, Bi, Ce, Pr, Sm, Eu, Gd, Tb, and Er (specifically, for example, NiO, $Cr_2O_3$), preferably oxides of at least one element selected from the group consisting of Ti, V, Cr, Mn, Fe, Co, Ni, Zn, Y, Zr, Sn, Sb, Bi, Ce, Pr, Sm, Eu, Gd, and Tb, and more preferably oxides of at least one element selected from the group consisting of Ti, V, Cr, Mn, Fe, Co, Ni, Zn, Y, Zr, Sn, Sb, Bi, Ce, Sm, Eu, Gd, and Tb. A zirconia pre-sintered body of the present invention may be one that does not comprise erbium oxide ($Er_2O_3$). Examples of the complex pigments include $(Zr,V)O_2$, $Fe(Fe,Cr)_2O_4$, $(Ni,Co,Fe)(Fe,Cr)_2O_4 \cdot ZrSiO_4$, and $(Co,Zn)Al_2O_4$. Examples of the fluorescent agents include $Y_2SiO_5$:Ce, $Y_2SiO_5$:Tb, $(Y,Gd,Eu)BO_3$, $Y_2O_3$:Eu, YAG:Ce, $ZnGa_2O_4$:Zn, and $BaMgAl_{10}O_{17}$:Eu.

A zirconia pre-sintered body of the present invention can be fabricated by firing (i.e., pre-sintering) a zirconia molded body formed from a raw material powder containing zirconia particles and a stabilizer at a temperature that does not sinter the zirconia particles. A zirconia molded body of the present invention comprises zirconia and a stabilizer capable of inhibiting a phase transformation of zirconia, and at least a part of the stabilizer is undissolved in zirconia as a solid solution. A zirconia molded body producing method of the present invention comprises a step of press forming a mixed powder comprising zirconia and the stabilizer at a pressure of 175 MPa or more to obtain a zirconia molded body (hereinafter also referred to as a "step of fabricating a zirconia molded body"). In view of obtaining a bulk density of 3.0 g/cm$^3$ or more not only in a zirconia pre-sintered body to be sintered to have a small thickness but also in a zirconia pre-sintered body to be sintered to have a large thickness (for example, a zirconia pre-sintered body having a thickness of 10 mm or more), the pressure is preferably 190 MPa or more, more preferably 200 MPa or more, even more preferably 215 MPa or more, and particularly preferably 220 MPa or more. Press molding at the above pressures can increase the bulk density of the zirconia molded body irrespective of the thickness (and thus the zirconia pre-sintered body obtained). The upper limit of the pressure in press molding the mixed powder is not particularly limited, and may be 600 MPa or less, 500 MPa or less, or 400 MPa or less. An excessively high pressure might cause occurrence of a crack in removing the molded body from a mold. In the present specification, the pressure of 175 MPa or more refers to the maximum pressure in press molding. By pre-sintering the zirconia molded body at 800 to 1,200°C, a zirconia pre-sintered body of the present invention can be obtained. The pre-sintering temperature is not particularly limited, and may be 850°C or more, 900°C or more, or 950°C or more. In addition, the pre-sintering temperature may be 1,150°C or less, 1,100°C or less, or 1,050°C or less. Commercially available products as conventionally known zirconia powders for use in zirconia molded body producing methods include yttria-stabilized tetragonal zirconia polycrystal that is predominantly tetragonal (Y-TZP, yttria content: 3 mol%, TZ-3Y grade or trade name "Zpex", manufactured by Tosoh Corporation), partially stabilized zirconia that is predominantly tetragonal and cubic (PSZ, yttria content: 5 to 5.3 mol%, TZ-5Y grade or trade name "Zpex Smile", manufactured by Tosoh Corporation), and partially stabilized zirconia (PSZ) that is predominantly cubic. In a zirconia molded body producing method of the present invention, it is preferable to use a zirconia powder that is predominantly monoclinic. A stabilizer used in the zirconia molded body producing method of the present invention is the same as that in the zirconia pre-sintered body mentioned above.

The following describes in detail a preferred example of a zirconia pre-sintered body producing method of the present invention. First, a raw material powder of a zirconia molded body is produced. A predominantly monoclinic zirconia powder and a stabilizer powder (for example, a yttria powder) are used to make a mixture of a desired stabilizer (for example, yttria) content. In a zirconia pre-sintered body producing method of the present invention, a predominantly monoclinic zirconia powder is preferably used. Then, the mixture is added to water to prepare a slurry, and the slurry was pulverized and mixed wet with a ball mill until the desired particle diameter (for example, an average particle diameter of 0.15 μm or less) is achieved. After pulverization, the slurry is dried to granulate, using a spray dryer. The resulting powder is then fired into a powder (primary powder) at a temperature (for example, 800 to 1,200°C) that does not sinter the zirconia particles. A pigment may be added to the primary powder. Then, the primary powder is added to water to prepare a slurry, and the slurry is pulverized and mixed wet with a ball mill until the desired particle diameter (for example, an average particle diameter of 0.13 μm or less) is achieved. After pulverization, an additive, such as a binder, is optionally added to the slurry, and the slurry is dried with a spray dryer to prepare a mixed powder (secondary powder). The secondary powder is charged into a predetermined die, and an upper surface of the secondary powder is leveled to be flat. With an upper die set on the secondary powder, the secondary powder is press molded using a uniaxial pressing machine to obtain a zirconia molded body. As described above, the pressure for press molding the mixed powder needs to be 175 MPa or more. Press molding may be performed in multiple stages or in a single stage (only once) as long as the pressure is 175 MPa or more. In view of easy production and industrial advantages, press-molding may be performed in a single stage. The obtained zirconia molded body may or may not be further subjected to molding by cold isostatic press (CIP). A preferred embodiment is a zirconia pre-sintered body producing method comprising: a step of press molding a mixed powder containing zirconia and a stabilizer capable of inhibiting a phase transformation of zirconia at a pressure of 175 MPa or more to obtain a zirconia molded body; and a step of firing the obtained zirconia molded body at 800 to 1,200°C, and at least a part of the stabilizer is undissolved in zirconia as a solid solution. Another preferred embodiment is a zirconia pre-sintered body producing method not comprising: a step of CIP molding.

In the case where a zirconia pre-sintered body having a multilayer structure is produced as described below, the primary powder may be divided into at least two (preferably four) groups in the above producing method in order to make a zirconia molded body have a multilayer structure. The following example divides the primary powder into four groups. A pigment is added to each of first to fourth powders. A pigment may be added to only one or some of the powder groups so that the pigment content will be different for the different powder groups. After adding the pigment, each powder is added to water to prepare a slurry, and the slurry was pulverized and mixed wet with a ball mill until the desired particle diameter is achieved. After pulverization, an additive, such as a binder, is optionally added to the slurry, and the slurry is dried with a spray dryer to produce secondary powders of first to fourth powders. The secondary powder of first powder is charged into a predetermined die, and an upper surface of the first powder is leveled to be flat. Subsequently, the second powder is charged onto the first powder, and an upper surface of the second powder is leveled to be flat. Similarly, the third powder is charged onto the second powder, and an upper surface of the third powder is leveled to be flat. Furthermore, the fourth powder is charged onto the third powder, and an upper surface of the fourth powder is leveled to be flat. Finally, with an upper die set on the powders, the secondary powders of four types (mixed powders) are subjected to press molding using a uniaxial pressing machine. As described above, the pressure for pressing the mixed powders needs to be 175 MPa or more. The obtained zirconia molded body of a four-layer structure may or may not be further subjected to CIP molding.

Next, the zirconia molded body thus obtained is pre-sintered to obtain a zirconia molded body. In order to ensure formation of a block, the pre-sintering temperature for pre-sintering a zirconia molded body of the present invention is, for example, preferably 800°C or more, more preferably 900°C or more, and even more preferably 950°C or more. In addition, for improved dimensional accuracy, the pre-sintering temperature is, for example, preferably 1,200°C or less, more preferably 1,150°C or less, and even more preferably 1,100°C or less. That is, the pre-sintering temperature is preferably 800°C to 1,200°C in a zirconia pre-sintered body producing method of the present invention. Pre-sintering should not drive the dissolution of the stabilizer as a solid solution with the pre-sintering temperature falling in this range.

A zirconia pre-sintered body of the present invention can be suitably used as a dental material. For example, the zirconia pre-sintered body, as the dental material, may be disc-shaped (circular disc) or block-shaped (cuboidal), or may have a shape of a dental product (for example, a shape of a crown). The zirconia pre-sintered body of the present invention is also inclusive of a dental product (for example, a crown-shaped prosthesis) made from a pre-sintered zirconia disc by processing with a CAD/CAM (Computer-Aided Design/Computer-Aided Manufacturing) system.

A zirconia pre-sintered body of the present invention can be fabricated into a high-translucency sintered body even with a short firing time. A sintered body fabricated by firing a zirconia pre-sintered body of the present invention at a suitable firing temperature for a certain time period is denoted herein as a third sintered body. A sintered body fabricated by firing a zirconia pre-sintered body of the present invention at the suitable firing temperature for 120 minutes is denoted herein as a fourth sintered body. In a comparison of the translucency of the third sintered body and the fourth sintered body, the translucency of a third sintered body with 30-minute firing is preferably at least 85%, more preferably at least 90%, even more preferably at least 95% of the translucency of the fourth sintered body, and particularly preferably essentially the same as the translucency of the fourth sintered body. The thickness of the third sintered body and the fourth sintered body in translucency evaluation may be for example 1.2 mm. Further, the translucency of a third sintered body with 15-minute firing is preferably at least 85%, more preferably at least 90%, even more preferably at least 95% of the translucency of the fourth sintered body, and particularly preferably essentially the same as the translucency of the fourth sintered body. As hereinbefore described, a zirconia pre-sintered body of the present invention has the time advantage for firing. The suitable firing temperature can be defined by the following method. First, a zirconia pre-sintered body is fired at various temperatures for 120 minutes, and then both surfaces of the resulting zirconia sintered body are polished with #600 paper to obtain a specimen of the zirconia sintered body having a thickness of 1.2 mm. The appearance of the obtained specimen is visually inspected. The zirconia pre-sintered body can be regarded as having been sufficiently fired, based on the state of the specimen having transparency clear enough to view the background. Meanwhile, the zirconia pre-sintered body can be determined as having been insufficiently fired, based on the state of the specimen having low transparency or being clouded. According to this determination, the lowest temperature at which the zirconia pre-sintered body can be regarded as having been sufficiently fired is defined as the suitable firing temperature of the zirconia pre-sintered body.

By firing a zirconia pre-sintered body of the present invention, a zirconia sintered body having excellent translucency can be obtained irrespective of the thickness. In the step of firing (sintering) a zirconia pre-sintered body, a general dental porcelain firing furnace can be used. The dental porcelain firing furnace may be a commercially available product. Examples of such commercially available products include "Noritake Katana (registered trademark) F-1N" and "Noritake Katana (registered trademark) F-2" (both available from SK MEDICAL ELECTRONICS CO., LTD.). The firing temperature is not particularly limited, but is preferably 1,400 to 1,650°C. The firing time for firing a zirconia pre-sintered body of the present invention is not particularly limited, and may be within 60 minutes, within 45 minutes, or within 30 minutes. Firing in such a short time allows to obtain a zirconia sintered body having excellent translucency irrespective of the thickness.

A zirconia pre-sintered body of the present invention may have a structure having a single composition, or may have a multilayer structure in view of reproducing the shade suited for dental use. The multilayer structure is for example a structure in which respective contents of zirconia, a stabilizer, a pigment, and a component affecting the appearance of a zirconia sintered body vary for each layer. More specifically, in the case where a zirconia pre-sintered body of the present invention has a multilayer structure, it is preferable in the zirconia pre-sintered body of the present invention that:

L1 be 68.0 or more and 90.0 or less,
a1 be -3.0 or more and 4.5 or less,
b1 be 0.0 or more and 24.0 or less,
L2 be 60.0 or more and 85.0 or less,
a2 be -2.0 or more and 7.0 or less,
b2 be 4.0 or more and 28.0 or less,
L1 > L2,
a1 < a2, and
b1 < b2,

where (L1,a1,b1) represent values of (L*,a*,b*) of the L*a*b* color system after sintering as measured at a first point falling within an interval of a length from one end of the zirconia pre-sintered body to 25% of the entire length of a straight line extending along a first direction from one end to the other end of the zirconia pre-sintered body, and (L2,a2,b2) represent values of (L*,a*,b*) of the L*a*b* color system after sintering as measured at a second point falling within an interval of a length from the other end of the zirconia pre-sintered body to 25% of the entire length of the straight line, and that:

the values of (L*,a*,b*) of the L*a*b* color system after sintering show unchanging patterns of increase and decrease in a direction from the first point to the second point.

More preferably, L1 is 69.0 or more and 89.0 or less, a1 is -2.7 or more and 4.0 or less, b1 is 1.0 or more and 23.5 or less, L2 is 61.5 or more and 84.5 or less, a2 is -1.5 or more and 6.5 or less, and b2 is 5.5 or more and 26.0 or less.

Even more preferably, L1 is 70.0 or more and 87.0 or less, a1 is -2.5 or more and 3.7 or less, b1 is 2.0 or more and 23.0 or less, L2 is 63.0 or more and 84.0 or less, a2 is -1.2 or more and 6.0 or less, and b2 is 7.0 or more and 24.0 or less.

By satisfying these ranges, the zirconia pre-sintered body can match its color with the average shade of a natural tooth.

In addition, in the case where a zirconia pre-sintered body of the present invention has a multilayer structure, preferably, L1 - L2 is more than 0 and 12.0 or less, a2 - a1 is more than 0 and 6.0 or less, and b2 - b1 is more than 0 and 12.0 or less.

More preferably, L1 - L2 is more than 0 and 10.0 or less, a2 - a1 is more than 0 and 5.5 or less, and b2 - b1 is more than 0 and 11.0 or less.

Even more preferably, L1 - L2 is more than 0 and 8.0 or less, a2 - a1 is more than 0 and 5.0 or less, and b2 - b1 is more than 0 and 10.0 or less.

Particularly preferably, L1 - L2 is 1.0 or more and 7.0 or less, a2 - a1 is 0.5 or more and 3.0 or less, and b2 - b1 is 1.6 or more and 6.5 or less.

Most preferably, L1 - L2 is 1.5 or more and 6.4 or less, a2 - a1 is 0.8 or more and 2.6 or less, and b2 - b1 is 1.7 or more and 6.0 or less.

By satisfying these ranges, the zirconia pre-sintered body can more preferably reproduce the shade of a natural tooth.

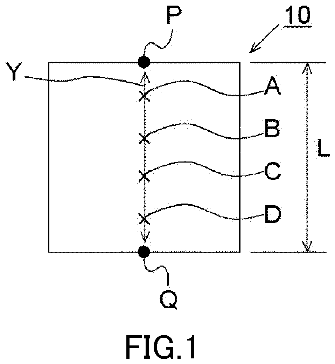

In the case where a zirconia pre-sintered body of the present invention has a multilayer structure, a zirconia sintered body obtained by firing the zirconia pre-sintered body preferably shows a color change from one end to the other end of the zirconia sintered body. This is described below with reference to FIG. 1, which is a schematic view of a zirconia sintered body. FIG. 1 shows a zirconia sintered body 10 with a straight line extending along a first direction Y from one end P to the other end Q. Preferably, the pattern of increase or decrease of L*, a*, and b* values does not change in the opposite direction. Specifically, when the L* value is in a pattern of decrease on a straight line from one end P to the other end Q, it is preferable that there exist no interval in which the L* value essentially increases. For example, referring to FIG. 1 showing first point A and second point D on a straight line connecting one end P to the other end Q, it is preferable that there exist no interval in which the L* value increases by 1 or more, more preferably 0.5 or more when the L* value is in a pattern of decrease from first point A to second point D on a straight line connecting first point A and second point D. When the a* value is in a pattern of increase on a straight line from one end P to the other end Q, it is preferable that there exist no interval in which the a* value essentially decreases. For example, when the a* value is in a pattern of increase from first point A to second point D on a straight line connecting first point A and second point D, it is preferable that there exist no interval in which the a* value decreases by 1 or more, more preferably 0.5 or more. When the b* value is in a pattern of increase on a straight line from one end P to the other end Q, it is preferable that there exist no interval in which the b* value essentially decreases. For example, when the b* value is in a pattern of increase from first point A to second point D on a straight line connecting first point A and second point D, it is preferable that there exist no interval in which the b* value decreases by 1 or more, more preferably 0.5 or more.

Concerning the direction of color change of the zirconia sintered body 10, it is preferable that the a* and b* values show a pattern of increase from one end P to the other end Q when the L* value is in a pattern of decrease in this direction. For example, the color changes from white to pale yellow, pale orange, or pale brown from one end P to the other end Q.

In the zirconia sintered body 10 of FIG. 1, a third point B is a point lying between first point A and second point D on a straight line connecting one end P to the other end Q. When the values of (L*,a*,b*) of the L*a*b* color system at third point B are (L3,a3,b3), it is preferable that L3 be 66.0 or more and 89.0 or less, a3 be -2.5 or more and 6.0 or less, b3 be 1.5 or more and 25.0 or less, L1 > L3 > L2, a1 < a3 < a2, and b1 < b3 < b2.

A fourth point C is a point lying between third point B and second point D. When the values of (L*,a*,b*) of the L*a*b* color system at the fourth point are (L4,a4,b4), it is preferable that L4 be 62.0 or more and 86.0 or less, a4 be -2.2 or more and 7.0 or less, b4 be 3.5 or more and 27.0 or less, L1 > L3 > L4 > L2, a1 < a3 < a4 < a2, and b1 < b3 < b4 < b2.

For the measured values (L*,a*,b*) in the multilayer structure, the individual zirconia sintered body produced from each layer was fabricated into a disc plate measuring 14 mm in diameter and 1.2 mm in thickness (both surfaces were polished, #600), and measured against a white background with a spectrophotometer CM-3610A, manufactured by Konica Minolta Inc. (D65 illuminant, measurement mode SCI, measurement area Ø:illumination area Ø = 8 mm:11 mm).

In the zirconia sintered body 10 of FIG. 1, it is preferable that the first point A lie within an interval of a length from one end P to 25% of the length between one end P and the other end Q (hereinafter, referred to as "entire length"). Preferably, the third point B lies within an interval distance away from one end P by a distance of 30% of the entire length and extending no further than 70% of the entire length relative to one end P. For example, the third point B may be distance away from one end P by a distance of 45% of the entire length. Preferably, the second point D lies within an interval of a length of 25% of the entire length from the other end Q. Preferably, the fourth point C lies within an interval distance away from the other end Q by a distance of 30% of the entire length and extending no further than 70% of the entire length relative to the other end Q. For example, the fourth point C may be distance away from the other end Q by a distance of 45% of the entire length (i.e., 55% of the entire length from one end P).

Concerning the foregoing descriptions given with reference to the schematic view shown in FIG. 1, it is preferable in the present invention that "one end" and "other end" refer to a point at the cut end and a point at the base end of a zirconia pre-sintered body or a sintered body thereof, for example, when the zirconia pre-sintered body or the sintered body has a crown shape. The point may be a point on the end surface, or a point on a cross section. A point falling within an interval of a length of 25% of the entire length from one end or the other end is, for example, a point that is away from one end or the other end by a distance corresponding to 10% of the height of the crown.

When a zirconia pre-sintered body of the present invention is disc-shaped, or has a shape of a hexahedron such as a cuboid, "one end" and "other end" preferably refer to points on the top surface and bottom surface (base). The point may be a point on the end surface, or a point on a cross section. A point falling within an interval of a length of 25% of the entire length from one end or the other end is, for example, a point that is away from one end or the other end by a distance corresponding to 10% of the thickness of the hexahedron or disc.

In the present invention, "first direction from one end to the other end" means a direction in which the color changes. For example, "first direction" is preferably the direction of powder lamination in the producing method described later. For example, when the zirconia pre-sintered body has a crown shape, "first direction" is preferably a direction connecting the cut end and the base end.

A zirconia sintered body obtained after firing of the zirconia pre-sintered body of the present invention can be suitably used as a dental product. Examples of such a dental product include copings, frameworks, crowns, crown bridges, abutments, implants, implant screws, implant fixtures, implant bridges, implant bars, brackets, denture bases, inlays, onlays, orthodontic wires, and laminate veneers. These may be produced by selecting methods that are suited for their intended use. For example, a dental product can be obtained by sintering a zirconia pre-sintered body of the present invention after milling. Preferably, the milling process uses a CAD/CAM system.

The present invention encompasses combinations of the foregoing features, provided that such combinations made in various forms within the technical idea of the present invention can produce the effects of the present invention.

EXAMPLES

The following describes the present invention in greater detail by way of Examples. It should be noted that the present invention is in no way limited by the following Examples, and various changes may be made by a person with ordinary skill in the art within the technical idea of the present invention.

[Fabrication of zirconia pre-sintered body]

In Examples and Comparative Examples, zirconia pre-sintered bodies were fabricated using the following procedures.

(Examples 1 to 6 and Comparative Example 1)

First, mixtures containing yttria in the amounts shown in Table 1 were prepared using a zirconia powder in which about 100% is monoclinic and a yttria powder. Each mixture was added to water to prepare a slurry, and pulverized and mixed wet with a ball mill until an average particle diameter of 0.15 $\mu$m or less was achieved. After pulverization, the slurry was dried with a spray dryer, and the resulting powder was fired at 950°C for 2 hours to prepare a primary powder. The average particle diameter can be determined by using a laser diffraction scattering method. Specifically, for the measurement using a laser diffraction scattering method, for example, a laser diffraction particle diameter distribution analyzer (SALD-2300, manufactured by Shimadzu Corporation) may be used with a 0.2% sodium hexametaphosphate aqueous solution used as dispersion medium.

Water was added to each of the obtained primary powders to prepare slurries, and the slurries were pulverized and mixed wet with a ball mill until an average particle diameter of 0.13 $\mu$m or less was achieved. After pulverization, a binder was added to each of the slurries and then the slurries were dried with a spray dryer to prepare a mixed powder (secondary powder).

Next, the secondary powder was charged in an amount so as to have a predetermined thickness into a die having an inner size of 24 mm $\times$ 19 mm, and an upper surface of the secondary powder was leveled to be flat. Finally, with an upper die set on the secondary powder, the secondary powder was press molded using a uniaxial pressing machine at a surface pressure shown in Table 1 for 90 seconds to obtain a zirconia molded body.

The obtained zirconia molded body was fired at 1,000°C for 2 hours to fabricate a zirconia pre-sintered body.

(Comparative Example 2)

The zirconia molded body obtained in the same manner as in Example 2 except for the pressing pressure set at 30 MPa in press molding was further subjected to CIP molding at 170 MPa for 5 minutes, and then was fired at 1,000°C for 2 hours to fabricate a zirconia pre-sintered body.

(Comparative Examples 3 to 6)

Zirconia pre-sintered bodies were fabricated in the same manner as in Example 1, except that "Zpex (registered trademark)" (predominantly tetragonal) manufactured by Tosoh Corporation was used as the secondary powder in Comparative Examples 3 and 4, and that "Zpex Smile (registered trademark)" (predominantly tetragonal and cubic: the sum of the tetragonal system and the cubic system is about 90%) manufactured by Tosoh Corporation was used as the secondary powder in Comparative Examples 5 and 6.

[Measurement of percentage presence $f_y$ of undissolved yttria]

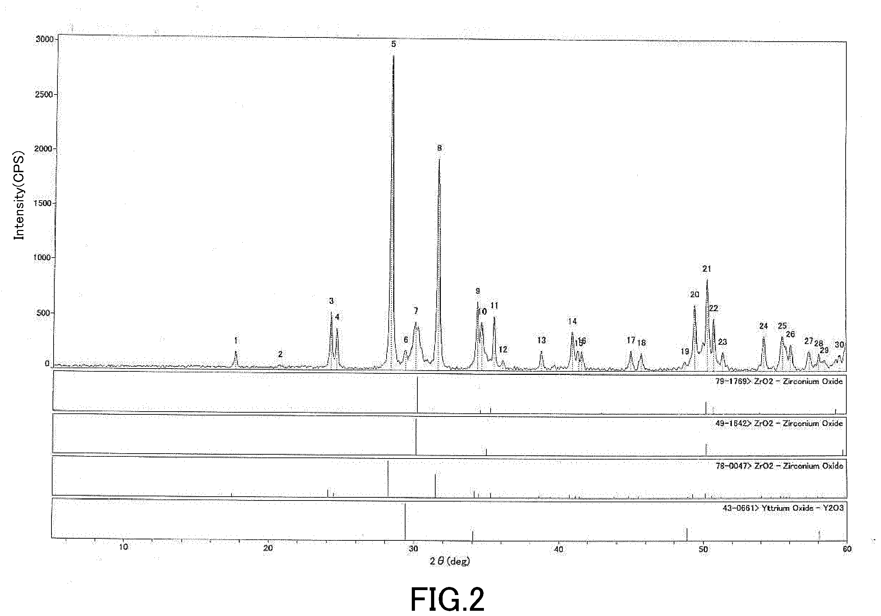

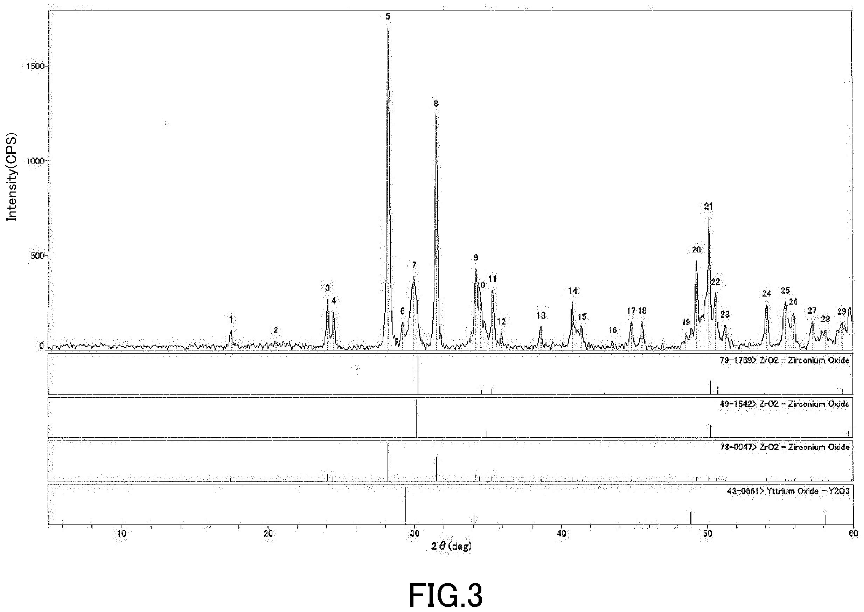

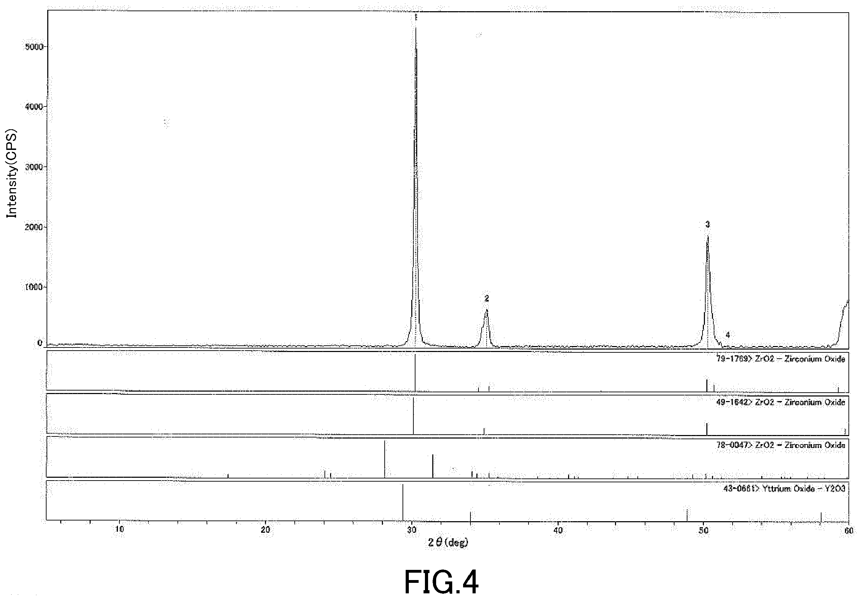

The XRD patterns were measured using CuK$\alpha$ rays on the zirconia pre-sintered bodies of Examples 1 to 6 and Comparative Examples 1 to 6 to calculate the percentage presence $f_y$. Table 1 shows the measurement results of the percentage presence $f_y$. FIG. 2 shows the XRD pattern of the zirconia pre-sintered body fabricated in Example 1. FIG. 3 shows the XRD pattern of the zirconia pre-sintered body fabricated in Example 3. FIG. 4 shows the XRD pattern of the zirconia pre-sintered body fabricated in Comparative Example 5.

As shown in FIG. 4, the zirconia pre-sintered body of Comparative Example 5 showed no peak attributed to the monoclinic crystal system of zirconia. A peak attributed to yttria was also not observable. The Comparative Examples 3, 4, and 6 produced the same results. In contrast, as shown in FIGS. 2 and 3, the zirconia pre-sintered bodies of Examples 1 and 3 showed peaks attributed to the monoclinic, tetragonal, and cubic crystal systems of zirconia, and the peak intensity was the highest for the monoclinic crystals of zirconia (peak numbers 5 and 8 in FIGS. 2 and 3). The same results were obtained in Examples 2 and 4 to 6. The zirconia pre-sintered bodies of the Examples all had peaks attributed to yttria near $2\theta = 29.4°$ (peak number 6 in FIGS. 2 and 3). From the above results, it is considered that a part of yttria was present in an undissolved form as a solid solution in the zirconia pre-sintered bodies of Examples 1 to 6 and that all parts of yttria were dissolved in zirconia as a solid solution in the zirconia pre-sintered bodies of Comparative Examples 1 to 6.

[Measurement of bulk density of zirconia pre-sintered body]

With respect to each of the zirconia pre-sintered bodies of Examples 1 to 6 and Comparative Examples 1 to 6, a pre-sintered body having a thickness of less than 10 mm and a pre-sintered body having a thickness of 10 mm or more were fabricated by adjusting the charging amount of the secondary powder in producing the zirconia molded body. The bulk density was measured on each of the pre-sintered bodies by the Archimedes method. Specifically, respective pre-sintered bodies having a thickness of 2 mm and a thickness of 14 mm were fabricated. A density measurement kit (ML-DNY-43) was attached to an electronic balance (ML204/02) manufactured by Mettler Toledo, and the bulk density was measured by the Archimedes method from the measured mass of the specimen in air and the measured mass of the specimen hanging in water. The measurement results are shown in Table 1.

[Measurement of translucency of zirconia pre-sintered body after firing]

With respect to each of the zirconia pre-sintered bodies of Examples 1 to 6 and Comparative Examples 1 to 6, a pre-sintered body having a thickness of 1 mm after firing and a pre-sintered body having a thickness of 10 mm after firing were fabricated by adjusting the charging amount of the secondary powder in producing the zirconia molded body, and were fired at the firing temperatures and the firing times described in Table 1 with an electromagnetic induction electric furnace in which SiC is used as the heating body. Thus, a first sintered body having a thickness of 1 mm and a second sintered body having a thickness of 10 mm were obtained. The firing time indicates the total time from when firing started to when the temperature reached 800°C due to cooling after reaching the maximum temperature in Table 1. The sintered bodies obtained were each polished to have a thickness of 0.5 mm. Thus, translucent specimens were obtained. The final finishing was performed with #2000 coated abrasive. The translucency of zirconia pre-sintered body after firing was calculated using the L* value of the luminance (color space) of the L*a*b* color system (JIS Z 8781-4:2013, Color measurements-Section 4: CIE 1976 L*a*b*color space) measured with a dental colorimeter (7 band LED light source, Crystaleye manufactured by Olympus Corporation). The specimen was measured for first L* value-an L* value measured against a white background, and second L* value-an L* value measured for the same specimen against a black background. The value ($\Delta$L*) obtained by subtracting the second L* value from the first L* value was determined as a numerical value indicating the translucency. In addition, the ratio of the translucency of the second sintered body (second translucency $\Delta L_2$*) to the translucency of the first sintered body (first translucency $\Delta L_1$*) was calculated using the numerical value. The measurement results are shown in Table 1.

[Table 1]

| | Ex. 1 | Ex. 2 | Ex. 3 | Ex. 4 | Ex. 5 | Ex. 6 | Com. Ex. 1 | Com. Ex. 2 | Com. Ex. 3 | Com. Ex. 4 | Com. Ex. 5 | Com. Ex. 6 |
|---|---|---|---|---|---|---|---|---|---|---|---|---|
| Yttria content (mol%) | 4 | 5 | 6 | 5 | 5 | 5 | 5 | 5 | 3 | 3 | 5.3 | 5.3 |
| Pressing pressure (MPa) | 200 | 200 | 200 | 175 | 225 | 200 | 150 | [1]30, 170 | 200 | 200 | 200 | 200 |
| Percentage presence $f_y$ of undissolved yttria (%) | 2.0 | 3.3 | 3.7 | 3.3 | 3.2 | 3.3 | 3.2 | 3.3 | 0.0 | 0.0 | 0.0 | 0.0 |
| Bulk density of pre-sintered body having thickness of 2 mm (g/cm$^3$) | 3.0 | 3.1 | 3.1 | 3.1 | 3.1 | 3.1 | 3.0 | 3.0 | 2.9 | 2.9 | 3.0 | 2.9 |
| Bulk density of pre-sintered body having thickness of 14 mm (g/cm$^3$) | 3.0 | 3.1 | 3.0 | 3.1 | 3.1 | 3.1 | 2.7 | 2.9 | 2.9 | 3.0 | 2.8 | 3.0 |
| Firing temperature in fabricating sintered body (°C) | 1515 | 1560 | 1560 | 1560 | 1560 | 1560 | 1560 | 1560 | 1515 | 1560 | 1515 | 1560 |
| Firing time in fabricating sintered body (min) | 30 | 30 | 30 | 30 | 30 | 60 | 30 | 30 | 30 | 30 | 30 | 30 |
| First translucency $\Delta L_1^*$ of sintered body having thickness of 1 mm | 12.5 | 16.8 | 17.2 | 16.9 | 16.7 | 16.8 | 16.9 | 16.8 | 12.8 | 13.1 | 14.8 | 16.2 |
| Second translucency $\Delta L_2^*$ of sintered body having thickness of 10 mm | 12.2 | 16.5 | 15.9 | 15.8 | 16.6 | 16.7 | 14.4 | 15.0 | 5.9 | 7.1 | 9.0 | 11.4 |
| Ratio of second translucency to first translucency (%) | 98 | 98 | 92 | 93 | 99 | 99 | 85 | 89 | 46 | 54 | 61 | 70 |
| 1) Primary pressing pressure and CIP molding pressing pressure are indicated in this order. | | | | | | | | | | | | |

As shown in Table 1, the pressing pressure in Comparative Example 1 is lower than the pressing pressure in Example 2, and the primary pressing pressure and the pressing pressure in CIP molding in Comparative Example 2 are both lower than the pressing pressure of Example 2. As a result, in Comparative Examples 1 and 2, the bulk density of the pre-sintered body having a thickness of 10 mm or more is less than 3.0 g/cm$^3$, the second translucency $\Delta L_2^*$ of the sintered body having a thickness of 10 mm is also low, and the ratio of the second translucency to the first translucency falls below 90%.

Further, in Comparative Examples 3 to 6 compared to Examples 1 and 2, the percentage presence $f_y$ of undissolved yttria is 0.0%. As a result, the second translucency $\Delta L_2^*$ of the sintered body having a thickness of 10 mm is significantly low, and the ratio of the second translucency to the first translucency is also extremely small.

Meanwhile, in the zirconia pre-sintered bodies of Examples 1 to 6, even the sintered body having a thickness of 10 mm exhibited high translucency equivalent to that of the sintered body having a thickness of 1 mm. This proves that the zirconia pre-sintered bodies can be fired in a short time and have excellent translucency after firing irrespective of the thickness.

The numeric ranges given in this specification should be construed such that all numerical values and ranges falling within the ranges specified herein are specifically recited in the specification, even in the absence of specific recitations.

INDUSTRIAL APPLICABILITY

The zirconia pre-sintered body of the present invention can be used as a dental material.

REFERENCE SIGNS LIST

10  zirconia sintered body
A   first point
B   third point
C   fourth point
D   second point
P   one end
Q   other end
L   entire length
Y   first direction

**Claims**

1. A method for producing a zirconia molded body, wherein the zirconia molded body comprises:

   zirconia; and
   a stabilizer capable of inhibiting a phase transformation of zirconia,
   at least a part of the stabilizer is undissolved in zirconia as a solid solution, and
   the method comprises
   press molding a mixed powder comprising zirconia and the stabilizer at a pressure of 175 MPa or more to obtain a zirconia molded body.

2. The method according to claim 1, wherein the stabilizer comprises yttria.

3. The method according to claim 2, wherein a percentage presence $f_y$ of the yttria undissolved in the zirconia as a solid solution as calculated from a mathematical expression (1) below is more than 0%,

$$f_y(\%) = \frac{I_y(111)}{I_y(111) + I_m(111) + I_m(11-1) + I_t(111) + I_c(111)} \times 100 \quad (1)$$

where $I_y$(111) represents a peak intensity of a (111) plane of yttria near $2\theta$ = 29° in an X-ray diffraction pattern using CuK$\alpha$ radiation, $I_m$(111) and $I_m$(11-1) represent peak intensities of a (111) plane and a (11-1) plane, respectively, of a monoclinic crystal system of zirconia in the X-ray diffraction pattern, $I_t$(111) represents a peak intensity of a (111) plane of a tetragonal crystal system of zirconia in the X-ray diffraction pattern, and $I_c$(111) represents a peak intensity of a (111) plane of a cubic crystal system of zirconia in the X-ray diffraction pattern.

4. The method according to any one of claims 1 to 3, wherein
the pressure in the press molding is 200 MPa or more.

5. The method according to any one of claims 1 to 4, wherein
the zirconia is predominantly monoclinic.

6. The method according to claim 5, wherein
a fraction $f_m$ of a monoclinic crystal system of zirconia is 55% or more as calculated from a mathematical expression (2) below,

$$f_m(\%) = \frac{I_m(111) + I_m(11-1)}{I_m(111) + I_m(11-1) + I_t(111) + I_c(111)} \times 100 \qquad (2)$$

where $I_m$(111) and $I_m$(11-1) represent peak intensities of a (111) plane a (11-1) plane, respectively, of a monoclinic crystal system of zirconia, $I_t$(111) represents a peak intensity of a (111) plane of a tetragonal crystal system of zirconia, and $I_c$(111) represents a peak intensity of a (111) plane of a cubic crystal system of zirconia.

7. A method for producing a zirconia pre-sintered body, the method comprising
firing a zirconia molded body obtained by the method according to any one of claims 1 to 6 at 800 to 1200°C.

8. A zirconia pre-sintered body comprising:

zirconia; and
a stabilizer capable of inhibiting a phase transformation of zirconia, wherein
with respect to a first sintered body having a thickness of 1 mm and a second sintered body having a thickness of 10 mm that are each fabricated by firing the zirconia pre-sintered body for 30 minutes, a ratio of a second translucency of a specimen having a thickness of 0.5 mm and fabricated from the second sintered body to a first translucency of a specimen having a thickness of 0.5 mm and fabricated from the first sintered body is 90% or more.

9. A zirconia pre-sintered body comprising:

zirconia; and
a stabilizer capable of inhibiting a phase transformation of zirconia, wherein
at least a part of the stabilizer is undissolved in zirconia as a solid solution, and
a bulk density measured by an Archimedes method using a specimen of a pre-sintered body having a thickness of 10 mm or more and fabricated from the zirconia pre-sintered body is 3.0 g/cm$^3$ or more.

10. The zirconia pre-sintered body according to claim 8 or 9, wherein
the stabilizer comprises yttria.

11. The zirconia pre-sintered body according to claim 10, wherein
a percentage presence $f_y$ of the yttria undissolved in the zirconia as a solid solution as calculated from a mathematical expression (1) below is more than 0%,

$$f_y(\%) = \frac{I_y(111)}{I_y(111) + I_m(111) + I_m(11-1) + I_t(111) + I_c(111)} \times 100 \qquad (1)$$

where $I_y$(111) represents a peak intensity of a (111) plane of yttria near $2\theta$ = 29° in an X-ray diffraction pattern using CuK$\alpha$ radiation, $I_m$(111) and $I_m$(11-1) represent peak intensities of a (111) plane and a (11-1) plane, respectively, of a monoclinic crystal system of zirconia in the X-ray diffraction pattern, $I_t(111)$ represents a peak intensity of a (111) plane of a tetragonal crystal system of zirconia in the X-ray diffraction pattern, and $I_c(111)$ represents a peak intensity of a (111) plane of a cubic crystal system of zirconia in the X-ray diffraction pattern.

12. A dental material comprising the zirconia pre-sintered body according to any one of claims 8 to 11.

13. The dental material according to claim 12, being disc-shaped or block-shaped.

FIG.1

FIG.2

FIG.3

FIG.4

## INTERNATIONAL SEARCH REPORT

| International application No. |
| --- |
| PCT/JP2020/017773 |

A.   CLASSIFICATION OF SUBJECT MATTER
C04B 35/48(2006.01)i; A61K 6/80(2020.01)i; A61K 6/84(2020.01)i
FI: C04B35/48; A61K6/80; A61K6/84

According to International Patent Classification (IPC) or to both national classification and IPC

B.   FIELDS SEARCHED

Minimum documentation searched (classification system followed by classification symbols)
C04B35/48; A61K6/80; A61K6/84

Documentation searched other than minimum documentation to the extent that such documents are included in the fields searched
Published examined utility model applications of Japan          1922–1996
Published unexamined utility model applications of Japan        1971–2020
Registered utility model specifications of Japan                1996–2020
Published registered utility model applications of Japan        1994–2020

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
| --- | --- | --- |
| Y | WO 2018/056330 A1 (KURARAY NORITAKE DENTAL INC.) 29.03.2018 (2018-03-29) paragraphs [0063]-[0075], [0095]-[0096], [0116]-[0142], [0150] | 1-13 |
| Y | WO 2018/056331 A1 (KURARAY NORITAKE DENTAL INC.) 29.03.2018 (2018-03-29) paragraphs [0067]-[0076], [0080]-[0081], [0105]-[0110], [0127] | 1-13 |
| Y | JP 2016-60687 A (KURARAY NORITAKE DENTAL INC.) 25.04.2016 (2016-04-25) paragraphs [0013]-[0017], [0045] | 1-13 |
| A | JP 2011-73907 A (WORLD LAB INC.) 14.04.2011 (2011-04-14) entire text, all drawing | 1-13 |

☐  Further documents are listed in the continuation of Box C.        ☒  See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 03 July 2020 (03.07.2020) | 14 July 2020 (14.07.2020) |

| Name and mailing address of the ISA/ Japan Patent Office 3-4-3, Kasumigaseki, Chiyoda-ku, Tokyo 100-8915, Japan | Authorized officer<br><br>Telephone No. |
| --- | --- |

Form PCT/ISA/210 (second sheet) (January 2015)

**INTERNATIONAL SEARCH REPORT**
Information on patent family members

International application No.

PCT/JP2020/017773

| Patent Documents referred in the Report | Publication Date | Patent Family | Publication Date |
|---|---|---|---|
| WO 2018/056330 A1 | 29 Mar. 2018 | EP 3517518 A1 paragraphs [0063]-[0073], [0093]-[0094], [O114]-[0140], [0148] CN 109689593 A KR 10-2019-0047701 A | |
| WO 2018/056331 A1 | 29 Mar. 2018 | US 2019/0231651 A1 paragraphs [0072]-[0079], [0083]-[0084], [0108]-[0113], [0130] EP 3517503 A1 CN 109790047 A KR 10-2019-0047702 A | |
| JP 2016-60687 A | 25 Apr. 2016 | (Family: none) | |
| JP 2011-73907 A | 14 Apr. 2011 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2016540562 A

- JP 2017128466 A